# EUROPÄISCHE PATENTSCHRIFT

(11) **EP 1 641 500 B1**
(45) Veröffentlichungstag und Bekanntmachung des Hinweises auf die Patenterteilung: **14.05.2008**
(21) Anmeldenummer: 04763139.5
(22) Anmeldetag: 08.07.2004
(51) Int. Cl.: A61L 27/06, A61L 27/50

(54) **ZEMENTIERBARE ENDOPROTHESEN**
CEMENTABLE ENDOPROSTHESES
ENDOPROTHESES CIMENTABLES

(30) Priorität: 08.07.2003 DE 10330793
(43) Veröffentlichungstag der Anmeldung: 05.04.2006
(73) Patentinhaber: POLIGRAT GMBH, 81829 München (DE)
(72) Erfinder: PIESSLINGER-SCHWEIGER, Siegfried, 85591 Vaterstetten (DE)
(74) Vertreter: Wibbelmann, Jobst
(86) Internationale Anmeldenummer: PCT/EP2004/007535
(87) Internationale Veröffentlichungsnummer: WO 2005/004941

(56) Entgegenhaltungen:
- EP-A- 0 249 650
- EP-A- 1 440 669
- WO-A-96/16611
- WO-A-03/044383
- US-A- 5 314 493
- US-A- 5 507 815
- VERDONSCHOT N ET AL: "Effects of prosthesis surface roughness on the failure process of cemented hip implants after stem-cement debonding." JOURNAL OF BIOMEDICAL MATERIALS RESEARCH. 15 DEC 1998, Bd. 42, Nr. 4, 15. Dezember 1998 (1998-12-15), Seiten 554-559, XP002307748 ISSN: 0021-9304

## Beschreibung

Die vorliegende Erfindung betrifft zementierbare metallische Endoprothesen mit zur Verankerung in der Zementierung dienenden Bereichen, die elektrochemisch oder chemisch poliert sind.

Die Verwendung von Prothesen wie z. B. Hüft-Endoprothesen oder Knie-Endoprothesen im Bereich der Implantationstechnik ist allgemein bekannt.

Metallische Endoprothesen beispielsweise für Gelenke werden wegen der hohen mechanischen Belastung und den Anforderungen an Korrosionsbeständigkeit und Gewebeverträglichkeit aus speziellen Legierungen wie beispielsweise Titanlegierungen, Edelstahllegierungen und Chrom-Kobalt-Basislegierungen hergestellt.

Die der Verankerung dienenden Bereiche der Endoprothesen werden bei der Implantation in den Knochen eingesetzt. Die Verankerung erfolgt dabei kraftschlüssig entweder direkt ohne Zementierung zwischen Endoprothese und Knochen oder durch Einzementierung der Endoprothese mittels Knochenzement. Das Ziel der Zementierung ist eine dauerhaft stabile und kraftschlüssige Verbindung ohne Relativbewegung und Spalten zwischen Knochen und Zementierung sowie zwischen Zementierung und Endoprothese.

Zur Zementierung werden unterschiedliche Zemente eingesetzt, die bestimmten Anforderungen an Festigkeit, Gewebeverträglichkeit, Haftvermögen und Röntgenkontrast genügen müssen. Um den Röntgenhontrast zu gewährleisten, werden spezielle Zuschlagstoffe wie Zirkonoxid oder Bariumsulfat verwendet. Teilbereiche der für die Verankerung vorgesehenen Prothesenoberflächen können sich dabei gevrollt oder ungewollt letztlich auch außerhalb der Zementierung befinden.

Die Implantationstechnik geht davon aus, dass der Knochenzement im gesamten Bereich der Zementierung und über die gesamte Lebensdauer eine stabile und kraftschlüssige Verankerung der Endoprothese im Knochen bewirkt, ohne dass an den Grenzflächen der Paarungen Knochen/Knochenzement und Knochenzement/Endoprothese Relativbewegungen oder Spalten auftreten.

Um eine möglichst intensive Verbindung zwischen Endoprothese und Knochenzement zu erreichen, werden die Oberflächen der Endoprothesen im Verankerungsbereich teilweise aufgeraut und strukturiert. Ein geeignetes und weiterhin gebräuchliches Verfahren hierfür ist das Strahlen. Vielfach sind Endoprothesen im Verankerungsbereich konisch geformt, um bei äußerer Belastung durch Keilwirkung den Anpressdruck im Zementmantel zu erhöhen. Sollte sich im postoperativen Verlauf die Verbindung zwischen Knochenzement und Endoprothese lockern (Debonding), geht man davon aus, dass durch leichtes axiales Nachsetzen der Endoprothese unter Belastung sich diese gegen den Zement neuerlich verkeilt und wieder kraftschlüssig fixiert wird. Dies erfolgt jedoch ausschließlich bei Druckbelastung. Eine Zugbelastung würde ein Lösen der Verbindung bewirken.

Die klinische Praxis zeigt jedoch, dass dauerhaft kraftschlüssige Verbindungen ohne Spalt und Relativbewegung zwischen Endoprothese und Zementierung vielfach nicht erreicht werden. Bedingt durch verschiedenste Einflüsse tritt vielmehr nach einiger Zeit eine teilweise oder vollständige postoperative Lockerung (Debonding) der Verbindung zwischen Endoprothese und Zementierung ein. Die Folge sind dauerhaft geringe periodische Gleitbewegungen mit Amplituden laut Fachliteratur unter 0,5 Millimeter bei gleichzeitig erhöhter Spannung im Zementmantel. Diese Relativbewegungen zwischen Endoprothese und Zementmantel verursachen Verschleißvorgänge mit der Folge der Generierung von Mikropartikeln durch abrasive Beanspruchung sowohl der Zement- als auch der Metalloberfläche sowie der Ausbildung von Spalten zwischen Endoprothesenoberfläche und Zementierung. Von der Zementierung werden vorzugsweise diejenigen Anteile verschlissen, die weicher sind als die Metalloberflächen, während Zuschlagstoffe wie Zirkonoxid mit einer höheren Härte als die Metalloberflächen zu deren abrasivem Abtrag führen. In der Folge entstehen Partikel in Submikrometergröße, die über den Spalt und Risse im Zementköcher an das umgebende Gewebe abgegeben werden und sich lokal anreichern können. Diese Partikel führen häufig zu Abwehrreaktionen des Organismus mit der Folge entzündlicher Prozesse und schließlich zu Knochenabbau und Lockerung der Endoprothese ("Wear Desease"). Es sind dies in der Regel sehr schmerzhafte Vorgänge, die letztlich eine erneute Operation notwendig machen.

Aus der EP 0 943 297 A1 (vergleichbar mit der US 6,383,228 B1) sind femorale Hüftgelenkprothesen bekannt, die nach einer bevorzugten Ausführungsform eine hochpolierte Oberfläche aufweisen sollen. Diese Endoprothesen sind so ausgestaltet, dass im implantierten Zustand der Knochenzement einer geringeren Belastung ausgesetzt ist, so dass insbesondere eine geringere Störung der Mikroverzahnung Zement/ Knochen-Grenzfläche beobachtet wird. Diese Druckschrift schlägt hierzu vor, dass man bei der Hüftgelenkprothese eine kragenlose Schulter am proximalen Ende und ein geradlinig zur Schulter zum distalen Ende verlaufender Schacht derart ausgestaltet ist, dass sich ein viereckförmiger Querschnitt ergibt. Bezüglich der vorzugsweise hochpoliert ausgestatteten Oberfläche führt das Dokument aus, dass dadurch die Haftung des Zements an der Zement/Implantat-Grenzfläche vermindert bzw. vermieden werden kann. Die Wirkung der Scherkräfte, die beim Absinken des Femurschaftes entlang des Schaftes auftreten oder die bei Torsion oder Biegung des Schaftes auftreten Scherkräfte im Zement sollen dadurch zusätzlich erheblich verringert werden. Diese Druckschrift beschreibt einen Zustand, bei dem keine kraftschlüssige Verbindung zwischen den Grenzflächen erfolgt, sondern diese mit möglichst geringer Reibung aneinander vorbeigleitet. Die Verankerung erfolgt durch Verkeilen, wobei der Prothesenschaft im Zementmantel in Druckrichtung wandert und eine irreversible Verkürzung des Oberschenkels und damit des Beines erfolgt. Davon unterscheiden sich die durch die Erfindung angesprochenen Endoprothesen schon darin, dass hinsichtlich der Verankerung der Prothese im wesentlichen ein stabiler Zustand erreicht werden soll und zwar unabhängig von der Form der Prothese. Eine solche Fixierung soll sowohl bei Zug- als auch Druckbeanspruchung wirksam sein und die Positionierung der Prothese möglichst sicherstellen.

Aus der US 5,456,723 A1 sind Prothesen bekannt, die ohne Zementierung implantiert werden sollen. Für diese Prothesen wird vorgeschlagen, dass sie einer beizenden Behandlung mit reduzierenden Säuren unterworfen werden. Dadurch wird ein sogenanntes "Pitting" (Lochbildung) erreicht, die das spätere Einwachsen der Knochensubstanz begünstigt. Solche Prothesen werden von der vorliegenden Erfindung nicht erfasst.

Auch die US 4,704,126 A1 beschreibt Prothesen, die ohne Zement direkt implantiert werden sollen. Die Oberfläche dieser Prothesen sollen so gestaltet sein, dass das Körpergewebe nicht einwächst und später ein Ausbau des Implantates leicht durchgeführt werden kann. Auch diese Druckschrift betrifft nicht solche Endoprothesen, die mit einem Zement implantiert werden.

Das Abriebproblem wird verstärkt, sobald zwischen Endoprothesenoberfläche und Zementmantel in der postoperativen Phase durch Verschleiß an der Grenzfläche zwischen Endoprothese und Zementmantel oder durch Zerrüttung innerhalb des Zementes Spalten entstehen. Darin eindringende Körperflüssigkeit kann innerhalb des Spaltes an der Metalloberfläche Korrosion auslösen (Spaltkorrosion), die zur verstärkten Abgabe von Metallionen an den Organismus führt. Sowohl bei Edelstahl- als auch bei Titanlegierungen begünstigt ein lokaler mechanischer Verschleiß zusätzlich den Korrosionsangriff auf diese Bereiche.

Die Aufgabe der vorliegenden Erfindung ist die Bereitstellung von einer metallischen Oberfläche bei metallischen Endoprothesen, bei denen auch nach vollständiger oder teilweiser postoperativer Lockerung der Zementierungsverankerung (Debonding) und bei ständigen Relativbewegungen zwischen Endoprothesenoberfläche und Zementierung eine Partikelgeneration im Vergleich zu handelsüblichen Endoprothesen so stark vermindert ist, dass Wear Disease vermieden wird.

Diese Aufgabe wird durch einen Verbund aus einer Endoprothese und einer darauf aufgebrachten Zementschicht wie in Anspruch 1 angegeben gelöst. Die erfindungsgemäßen Endoprothesen sind dadurch gekennzeichnet, dass die der Verankerung in der Zementierung dienenden Bereiche der Endoprothesenoberflächen so ausgestaltet sind, dass auch nach postoperativer Lockerung des Verbundes und bei ständigen Relativbewegungen zwischen Endoprothesenoberfläche und Zementierung eine Partikelgeneration, falls nicht ganz vermieden, so jedoch zumindest auf ein unkritisches Maß reduziert wird.

Dies wird dadurch erreicht, dass zumindest der der Verankerung in der Zementierung dienende Oberflächenbereich einer metallischen Endoprothese ganz oder teilweise elektrochemisch oder chemisch poliert wurde.

Die vorliegende Erfindung geht von der Idee aus, dass einerseits zwischen der Endoprothese und dem Zement durch die Makrostruktur der Endoprothese eine ausreichende Verzahnung erreicht wird (wodurch die Position der Prothese im Zementköcher im wesentlichen unabhängig von der Form der Prothese gehalten wird; Makrostruktur). Zum anderen wird aber eine Mikrorauheit angestrebt, die die Generierung von Partikeln, deren Größe im Nanobereich liegen kann, verhindern soll. Die Größe dieser Mikrorauheit liegt bei sehr kleinen Werten (kleiner als etwa 8 µm).

Makrorauheit wird in diesem Zusammenhang gemäß DIN 4760 als Gestaltsabweichungen im Bereich dritter und vierter Ordnung verstanden, Mikrorauheit als Gestaltsabweichungen fünfter und sechster Ordnung. Die erfindungsgemäße Endoprothese zeichnet sich also dadurch aus, dass sie nach der angegebenen DIN-Norm 4760 einmal eine Makrorauheit im Bereich dritter und vierter Ordnung und eine Mikrorauheit im Bereich fünfter und sechster Ordnung aufweist.

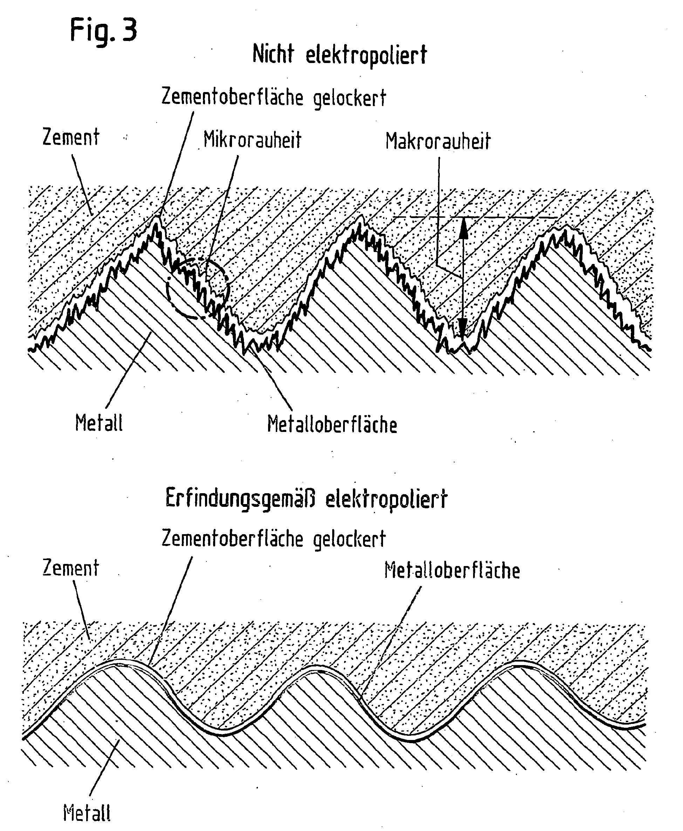

Phänomenologisch lässt sich die Erfindung anhand der Figur 3 erläutern. Die metallische Oberfläche einer Endoprothese, die üblicherweise einem Sandstrahiverfahren unterworfen wurde, weist eine nach DIN 4760 bestimmte Makrorauheit von größer als 20 µm (R_{z}-Wert) auf. Das Verhalten einer derart behandelten Endoprothese im implantierten Zustand wird im oberen Teil der Figur gezeigt. An der Grenzfläche Zement/Prothese liegt dann eine gewisse Mikrorauheit vor, die möglicherweise die Ursache für den unerwünschten Abrieb darstellt, oder diesen zumindest fördert. Durch das erfindungsgemäße Verfahren wird nun die metallische Oberfläche der Endoprothesen derart verändert, dass im Ergebnis eine deutliche Verminderung des unerwünschten Abriebs zu beobachten ist. Dies lässt sich phänomenologisch so beschreiben, dass einmal durch Verrundung der Rauhigkeitsspitzen die Makrorauheit soweit reduziert wird, dass ein Rauhigkeitswert erreicht wird, der etwa 10 bis 20 µm unterhalb des R_{z}-Wertes liegt, der nach Sandstrahlung der Endoprothese erreicht wird. Einen solchen Zustand erhält man beispielsweise mittels einer Elektropolitur, bei der ein Abtrag von der Oberfläche von etwa 10 bis 50 µm festgestellt wird. Die absolute Größe des Abtrags ist nicht kritisch, kann aber mit einfachen Mitteln in den gewünschten Grenzen eingestellt werden. Zugleich kommt es aber infolge der Elektropolitur zu einer Glättung der metallischen Oberfläche im Mikrorauheitsbereich (entsprechend R_{z} ≤ 0,5 µm), wobei Gestaitsabweichungen fünfter und sechster Ordnung, die einer Struktur dritter und vierter Ordnung überlagert ist, gemäß DIN 4762 und DIN 4763 messtechnisch schwer erfassbar und in Zahlen auszudrücken ist. Dieser Zustand ist schematisch in der unteren Hälfte der Figur gezeigt. Die Grenzfläche Zement/Prothese ist durch eine deutliche Glattheit gekennzeichnet, die aber nicht soweit geht, dass ein beträchtlicher Haftungsverlust an dieser Grenzfläche eintritt, wie er im Stand der Technik beispielsweise in der EP 0 943 297 A1 gefordert wird.

Umfangreiche Untersuchungen haben zu der überraschenden Erkenntnis geführt, dass die Entstehung von Partikeln weniger durch die Makrostruktur der Prothesenoberfläche beeinflusst wird als vielmehr durch deren Mikrostruktur und energetischen Zustand. Eine Makrostruktur, die zu einem Stützeffekt und damit zu einer besseren Übertragung und Verteilung der Kräfte zwischen Endoprothese und Zementmantel führt, hat nur bedingt Einfluss auf die Partikelgeneration. Es zeigte sich vielmehr überraschend, dass an mikroglatten Oberflächen, selbst wenn sie makroskopische Strukturen aufweisen, im Verankerungsbereich auch bei gelockertem Verbund und deutlicher Relativbewegung zwischen Endoprothese und Zementmantel die Bildung von Partikeln im Interface entscheidend verringert ist.

Die erfindungsgemäßen metallischen Endoprothesen weisen daher im Zementierungsbereich teilweise oder vollständig Oberflächen auf, die im Mikrobereich sehr glatt sind und ein niedriges Energiepotential aufweisen. Die Herstellung dieser Oberflächen erfolgt durch eine hochwertige elektrochemische oder chemische Politur der Metalloberflächen, wobei diese Bearbeitung bei den verwendeten Legierungen im Vergleich zu einer mechanischen Bearbeitung die Korrosionsbeständigkeit deutlich verbessert.

Gegenüber dem bisherigen Stand der Technik lässt der Einsatz der erfindungsgemäßen metallischen Endoprothesen bei Implantierung durch Zementierung eine wesentliche Reduzierung oder völlige Vermeidung postoperativer Komplikationen bedingt durch Partikelgeneration und verstärkte Korrosion an den Grenzflächen zwischen Endoprothese und Zementmantel erwarten.

Durch die elektrochemische oder chemische Politur der erfindungsgemäßen metallischen Endoprothesen im Bereich der späteren Zementierung, wird von der Oberfläche eine Werkstoffschicht definierter Dicke durch Auflösung des Metalls im Elektrolyten abgetragen. Dieser Abtrag bewirkt eine Einebnung und Glättung der Oberflächen im Mikrobereich. Gleichzeitig werden lokale Zug- und Druckspannungen sowie Werkstoffdefekte und Fremdpartikel entfernt, die aus den vorausgegangenen Bearbeitungsprozessen wie Strahlen herrühren und das Verschleißverhalten zusätzlich nachteilig beeinflussen (Dreikörperverschleiß).

Bei der Herstellung der erfindungsgemäßen metallischen Endoprothesen kommt der Qualität der elektrochemischen bzw. chemischen Politur der Oberflächen im Verankerungsbereich wesentliche Bedeutung zu. Es ist unbedingt erforderlich, dass eine homogene, geschlossene und mikroglatte Oberfläche ohne Korngrenzenangriff entsteht, um den Reibverschleiß zwischen Endoprothesenoberfläche und Zementmantel zu minimieren.

Die elektrochemische oder chemische Politur der Endoprothesenoberflächen erfolgt unter Verwendung moderner Elektropolierverfahren wie sie beispielsweise in der EP 0 249 650 beschrieben sind.

Zur Elektropolitur werden die zu behandelnden Oberflächen mit einem auf den jeweiligen Werkstoff abgestimmten Elektrolyten in Kontakt gebracht und anodisch in einem Gleichstromkreis geschaltet. Stromdichte, Elektrolyttemperatur und Behandlungszeit werden dabei auf das angestrebte Ergebnis hin optimiert und eingestellt.

Chemische Polierverfahren wie z. B. in der französischen Patentanmeldung 2,457,315 für die Behandlung von Edelstahloberflächen beschrieben, wirken ohne äußere Stromquelle durch chemische Reaktionen innerhalb der einzelnen Komponenten der Bäder. Zur Erzielung der der Erfindung zugrunde liegenden Qualität der Polierung ist eine sorgfältige Überwachung von Badzusammensetzung, Badbewegung, Konzentration des im Bad gelösten Metalls sowie der Temperatur erforderlich.

Nach der elektrochemischen oder chemischen Politur der Endoprothesen sind die Oberflächen sorgfältig zu waschen, um alle Rückstände von Chemikalien zu entfernen.

Die erfindungsgemäßen metallischen Endoprothesen bestehen aus Titan, Titanlegierungen, Edelstahllegierungen, Chrom-Kobalt-Basislegierungen oder Zirkonlegierungen. Bei den metallischen Endoprothesen gemäß der vorliegenden Erfindung handelt es sich bevorzugt um Implantate für den Bereich der Orthopädie Schulter-, Becken-, Hüft- und Kniegetenkprothesen.

Die erfindungsgemäßen metallischen Endoprothesen können über den gesamten Oberflächenbereich elektrochemisch oder chemisch poliert sein, zumindest aber sind die der Verankerung in der Zementierung dienenden Bereiche ganz oder teilweise elektrochemisch oder chemisch poliert.

Gemäß einer weiteren Ausführungsform kann die Korrosionsbeständigkeit passivierbarer Metalloberflächen zusätzlich verbessert werden, indem die Ausbildung der Passivschichten nach dem elektrochemischen oder chemischen Polieren durch eine geeignete Nachbehandlung wie Anodisieren oder Passivieren gezielt verstärkt wird.

Anodisieren eignet sich bevorzugt für Titan, Titanlegierungen sowie Zirkon. Der Anodisierungsschritt umfasst das Tauchen der zu behandelnden Oberflächen in einem speziellen sauren oder alkalischen Elektrolyten und die anodische Schaltung in einem Gleichstromkreis. Der Prozess setzt an der anodischen Oberfläche Sauerstoff frei, der mit dem Metall reagiert und eine dichte und korrosionsbeständige Oxidschicht erzeugt. Die Dicke der Oxidschichten hängt wesentlich von der angelegten elektrischen Spannung und der Behandlungszeit ab. Die anodisierten Oberflächen zeigen abhängig von der Dicke der Oxidschicht typische Farben durch Lichtinterferenz. Oberflächen von Zirkon und Zirkonlegierungen erhalten durch die hohe Härte der Oxidschichten einen zusätzlichen Verschleißschutz.

Ein zusätzlicher Passivierungsschritt wird bevorzugt bei Edelstahl, Nickelbasislegierungen und Chrom-Kobalt-Legierungen eingesetzt. Die Passivierbehandlung besteht meist in einer stromlosen Tauchbehandlung in Bädern. Diese können wässrige Lösungen oxidierender Medien wie Salpetersäure oder Wasserstoffperoxid enthalten oder wässrige Gemische von Chelat- und Komplexbildnern, wie sie aus der Literatur bekannt sind.

### Kurze Beschreibung der Figuren

Fig. 1 Rasterelektronenmikroskopische Aufnahmen der unbehandelten Vergleichsproben (Prüflos 1)
Fig. 2 Rasterelektronenmikroskopische Aufnahmen der erfindungsgemäß behandelten Proben (Prüflos 2)
Fig. 3 schematische Darstellung der Erfindung (unten) im Vergleich zum Stand der Technik (oben)

Die vorliegende Erfindung soll anhand von Beispielen näher erläutert werden.

Beispielhaft wurden Stiele von Hüftendoprothesen, welche für den zementierten Einsatz ausgelegt sind, elektrochemisch poliert und hinsichtlich des Abriebverhaltens mit unbehandelten, baugleichen Stielen verglichen.

### Durchführung

Fünf Prothesenstiele mit identischer Oberfläche, Werkstoffzusammensetzung und Design wurden von einem Hersteller direkt bezogen. Sie bestanden TiAI₆Nb₇, einer für diesen Einsatz gebräuchlichen Titanlegierung und wiesen eine durch Strahlen endbehandelte makroraue Oberfläche auf. Als Strahlgut waren Korund und Glaspartikel verwendet worden. Drei Stiele (Prüflos 1) verblieben im Anlieferungszustand ohne weitere Oberflächenbehandlung, während zwei Stiele (Prüflos 2) zusätzlich elektropoliert und anodisiert wurden. Die gestrahlten Oberflächen wiesen im Anlieferungszustand einen Rauhigkeitswert von Rz ca. 20 µm auf.

Zum Elektropolieren wurde der Elektrolyt E 392® der Firma POLIGRAT verwendet. Die Bearbeitung erfolgte mit einer Stromdichte von 2 A/dm² und einer Elektropolierdauer von 180 min.

Durch die Elektropolitur erfolgte ein Abtrag von ca. 30 µ, bis keine Reste von in die Oberfläche eingebettetem Strahlgut bzw. durch dieses verursachte Defekte mehr feststellbar waren. Anschließend wurden die elektropolierten Oberflächen in einem Elektrolyten bestehend aus 30 % Phosphorsäure und 70 % Wasser bei einer Spannung von 90 Volt und einer Behandlungszeit von 10 min. goldfarben anodisiert. Die Oberflächen der so behandelten Endoprothesenstiele wiesen einen Rauhigkeitswert von Rz ca. 8 µm auf und zeigten bei mikroskopischer Betrachtung eine homogene und geschlossene Struktur ohne Korngrenzenangriff.

Mit Hilfe eines speziellen Prüfmodells (Bader R., Steinhauser El, Guder S., Gregory J.K., Gradinger R., Mittelmeier W.: Modell zur in-vitro-Prüfung von Hüftendoprothesen-Stielen am Interface Implantatobernäche-Knochenzement. In: Eigenschaften und Prüftechniken mechanisch beanspruchter Implantate. DVM-Arheitskreis "Biowerkstoffe", DVM, Berlin (2002), pp. 165-174), wurde in der Grenzfläche zwischen Implantat oberfläche und Knochenzement das Abriebverhalten der Endoprothesenstiele analysiert und Abriebpartikel generiert.

Die Endoprothesenstiele wurden jeweils distal in Knochenzement eingebettet, wobei der verwendete Knochenzement Partikel aus Zirkonoxid als Zuschlagstoff zur Verbesserung des Röntgenkontrastes enthielt. Im Anschluss wurden zur Simulation des gelockerten Zustandes die Stiele aus dem Knochenzement entfernt und der Zementmantel standardisiert gespalten. Zur Generierung von Abriebpartikeln wurden unter trockenen Umgebungsbedingungen über einen weggeregelten Hydraulikzylinder zyklisch definierte Relativbewegungen mit Weglängen unter 0,5 mm über eine Dauer von 3 Mio. Lastwechseln in die Grenzflächen zwischen Endoprothesenstiel und Knochenzement eingeleitet. Mittels eines kraftgeregelten Hydraulikzylinders wurden über die gesamte Versuchsdauer definiert radiale Flächenpressungen zwischen Stiel und Zementköcher eingestellt.

### Ergebnis

Bei den unbehandelten Endoprothesenstielen des Prüfloses 1 zeigte sich an den beanspruchten Flächen eine Abnahme des Rz-Wertes von bis zu 2,5 µm, während diese bei den elektropolierten und anodisierten Prothesen des Prüfloses 2 nur bis maximal 1,0 µm betrug.

Makroskopisch zeigten sich bei den unbehandelten Stielen des Prüfloses 1 an den beanspruchten Arealen an den proximalen Enden der Stiele ventral- und dorsalseitig großflächig geglättete und polierte Oberflächen. An den elektropolierten Stielen des Prüfloses 2 zeigten sich deutlich geringere abrasive Veränderungen mit partiellem Abtrag der Anodisierungsschicht, insbesondere dorsalseitig.

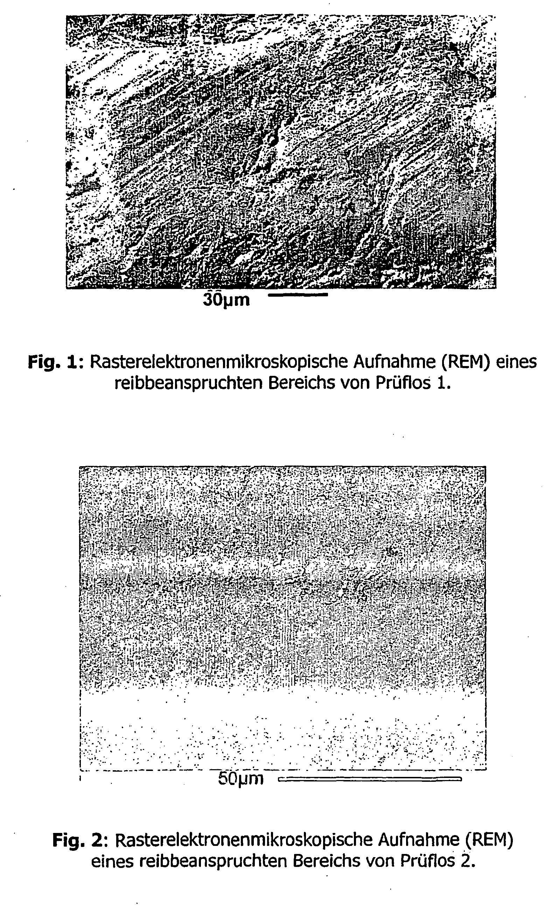

In rasterelektronenmikroskopischen Untersuchungen der Endoprothesenstiele zeigten sich an den unbehandelten Stielen des Prüfloses 1 flächig eingeebnete Bereiche mit abrasiven Verschleißspuren. Dabei deutet die deutliche Ausprägung der Verschleißspuren mit Riefen auf einen Dreikörperverschleiß, bedingt auch durch die dem Knochenzement beigefügten Partikel aus Zirkonoxid (Fig. 1). Auch an den behandelten Endoprothesenstielen des Prüfloses 2 fanden sich abrasive Veränderungen im Sinne eines Dreikörperverschleißes, jedoch deutlich weniger stark ausgeprägt (Fig. 2). Zwischen beiden Prüflosen waren deutliche Unterschiede hinsichtlich der Menge der durch Abrieb generierten Partikel zu beobachten.

Nach 3 Mio. Lastwechseln betrug der gravimetrisch ermittelte Materialabtrag (Knochenzement- und Metallpartikel) bei den rauen, unbehandelten Endoprothesenstielen des Prüfloses 1 im Mittel 92 mg/Stiel, wovon der Anteil an Metallpartikeln ca. 0,5 mg betrug. An den elektropolierten und anodisierten Endoprothesenstielen des Prüfloses 2 war der Materialabtrag pro Stiel jeweils deutlich geringer und lag unter der Erfassungsgrenze von 25 mg.

## Patentansprüche

1. Verbund aus einer metallischen Endoprothese aus Titan, Titanlegierung, Edelstahllegierung oder Chrom-Kobalt-Basislegierung und einer darauf aufgebrachten Zementschicht, **dadurch gekennzeichnet, dass** die mit einer Zementschicht verbundene Oberfläche der Endoprothese eine makrorauhe Oberfläche aufweist, die zusätzlich elektrochemisch oder chemisch poliert wurde, und die Oberfläche einen Rauhigkeitswert R_{z} (Makrorauheit) < 20 µm aufweist.

2. Verbund nach Anspruch 1, **dadurch gekennzeichnet, dass** die Makrorauheit der Oberfläche durch Strahlen erzeugt wurde.

3. Verbund nach Anspruch 1 oder 2, **dadurch gekennzeichnet, dass** nach der elektrochemischen Politur ein Rauhigkeitswert R_{z} (Makrorauheit) von < 15 µm der Oberfläche vorliegt.

4. Verbund nach einem der Ansprüche 1 bis 3, **dadurch gekennzeichnet, dass** die Oberfläche nach dem elektrochemischen oder chemischen Polieren zusätzlich anodisiert wurde.

5. Verbund nach einem der Ansprüche 1 bis 3, **dadurch gekennzeichnet, dass** Oberflächen, welche aus Edelstahllegierungen oder Chrom-Kobalt-Basislegierungen bestehen, nach dem elektrochemischen oder chemischen Polieren unter Verwendung von Chelatbildnern und/oder Zitronensäure zusätzlich passivlert wurden.

6. Verwendung einer metallischen Oberfläche bei metallischen Endoprothesen, die eine durch Strahlen und anschließende chemische oder elektrochemische Politur erhaltene makrorauhe Oberfläche mit einem Rauhigkeitswert R_{z} (Makrorauheit) < 20 µm aufweist, zur Vermeidung oder Verminderung von Abrieb an der Kontaktfläche Zement/Endoprothese.

7. Verwendung nach Anspruch 6, wobei die Oberfläche der Endoprothese einen Rauhigkeitswert R_{z} (Makrorauheit) < 15 µm aufweist.

## Claims

1. Composite structure comprised of a metallic endoprosthesis of titanium, titanium alloy, stainless steel alloy or chromium-cobalt master alloy and a cement layer applied thereto, **characterized in that** the surface of the endoprosthesis connected to a cement layer is a macrorough surface which was additionally electrochemically or chemically polished, and that the surface has a roughness value R_{z} (macroroughness) < 20 µm.

2. Composite structure according to claim 1, **characterised in that** the macroroughness of the surface was generated by blasting.

3. Composite structure according to claim 1 or 2, **characterized in that** after the electrochemical polishing there is a roughness value Rz (macroroughness) of < 15 µm of the surface.

4. Composite structure according to one of claims 1 to 3, **characterized in that** the surface was additionally anodized after it was electrochemically or chemically polished.

5. Composite structure according to one of claims 1 to 3, **characterized in that** surfaces consisting of stainless steel alloys or chromium-cobalt master alloys were additionally rendered passive by using chelate formers and/or citric acid, after they were electrochemically or chemically polished.

6. Use of a metallic surface with metallic endoprostheses, which has a macrorough surface having a roughness value R_{z} (macroroughness) < 20µm obtained by blasting and subsequent chemical or electrochemical polishing, in order to prevent or reduce abrasion on the contact surface cement/endoprosthesis.

7. Use according to claim 6, the surface of the endoprosthesis having a roughness value R_{z} (macroroughness) < 15 µm.

## Revendications

1. Combinaison d'une endoprothèse métallique constituée de titane, d'un alliage de titane, d'un alliage d'acier inoxydable ou d'un alliage de base cobalt-chrome, et d'une couche de ciment appliquée sur ladite endoprothèse, **caractérisée en que** la surface de l'endoprothèse liée à la couche de ciment présente une surface macro-rugueuse qui, de surcroît, a été polie de façon électrochimique ou chimique, et que ladite surface présente une valeur de rugosité R_{z} (macro-rugosité) inférieure à 20 µm.

2. Combinaison selon la revendication 1, **caractérisée en ce que** la macro-rugosité de la surface est obtenue par jet.

3. Combinaison selon la revendication 1 ou 2, **caractérisée en ce que**, après polissage électrochimique, la valeur de rugosité R_{z} (macro-rugosité) de la surface obtenue est inférieure à 15 µm.

4. Combinaison selon l'une des revendications 1 à 3, **caractérisée en ce que** la surface a, de surcroît, été anodisée après la passe de polissage électrochimique ou chimique.

5. Combinaison selon l'une des revendications 1 à 3, **caractérisée en ce que** les surfaces constituées d'alliages d'acier inoxydable ou d'alliages de base cobalt-chrome ont, après la passe de polissage électrochimique ou chimique, été de surcroît passivées par utilisation de chélateurs et/ou d'acide citrique.

6. Utilisation sur endoprothèses métalliques d'une surface métallique dont la macro-rugosité obtenue par jet suivi d'une passe de polissage chimique ou électrochimique présente une valeur de rugosité R_{z} (macro-rugosité) inférieure à 20 µm afin d'éviter ou de réduire l'usure par abrasion à la surface de contact ciment/endoprothèse.

7. Utilisation selon la revendication 6, la surface de l'endoprothèse présentant une valeur de rugosité R_{z} (macro-rugosité) inférieure à 15 *µ*m.
